# EUROPEAN PATENT APPLICATION

(11) **EP 4 257 120 A1**
(43) Date of publication of application: **11.10.2023**
(21) Application number: 20963980.6
(22) Date of filing: 04.12.2020
(51) Int. Cl.: A61K 9/19, A61K 31/675, A61K 47/26, A61K 47/36, A61P 25/08

(54) **FOSPHENYTOIN SODIUM SOLID COMPOSITION, LYOPHILIZATION METHOD, AND USE OF FOSPHENYTOIN SODIUM SOLID COMPOSITION**

(71) Applicant: Sichuan Credit Pharmaceutical Co., Ltd, Sichuan 646100 (CN)
(72) Inventor: CHEN, Gang, Luzhou, Sichuan 646100 (CN); CHEN, Gongzheng, Luzhou, Sichuan 646100 (CN); LIN, Song, New Haven, Connecticut 06510 (US); NELLAIAPPAN, Kaliappanadar, Wilmington, Massachusetts 01887 (US); JAVERI, Indu, Wilmington, Massachusetts 01887 (US)
(74) Representative: Bandpay & Greuter
(86) International application number: PCT/CN2020/133822
(87) International publication number: WO 2022/116134

(57) **Abstract**

The present disclosure relates to a fosphenytoin sodium solid composition, a lyophilization method of fosphenytoin sodium, and use of the fosphenytoin sodium solid composition. The fosphenytoin sodium solid composition comprises fosphenytoin sodium and at least one carbohydrate. The fosphenytoin sodium solid composition prepared in the present disclosure is stable and can be stored at room temperature. In addition, the lyophilization method for the fosphenytoin sodium is short in lyophilization time, the product obtained by the present method does not collapse, the reconstitution time is short, and the moisture content satisfies the quality requirements. The fosphenytoin sodium solid composition can be used for treatment of epilepsy or other convulsion states.

## Description

### Technical Field

The present invention relates to a fosphenytoin sodium solid composition, a lyophilization method, and use of the fosphenytoin sodium solid composition.

### Background

Fosphenytoin sodium, which is a phosphate prodrug of phenytoin, was approved by FDA of the US in September, 1996 for treating and controlling epilepsy and other types of convulsive states. Fosphenytoin sodium has no pharmacological activity before it is converted to phenytoin in the body. Therefore, its pharmacological effects are attributed to phenytoin.

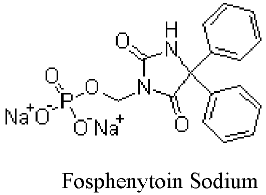

Fosphenytoin sodium was developed to replace phenytoin. Excessive propylene glycol and relatively high pH (12) in the formula of commercially available phenytoin sodium injections can cause severe pain at the administration site, hypotension, progressive limb ischemia distal to the infusion site, and other vascular complications, including "Purple Glove Syndrome". Fosphenytoin sodium can be applied to avoid phenytoin sodium-related problems.

Currently, the dosage form of commercially available fosphenytoin sodium is injection, which needs to be stored at 2-8°C and cannot be stored at room temperature for more than 48 hours. Fosphenytoin sodium injection is unstable at room temperature, and is prone to degradation and thus causes degradation impurities, such as diphenylglycine, diphenyl hydantoic acid and phenytoin, and thus affecting effectiveness of its clinical application. In addition, the storage condition of 2°C to 8°C increases the transportation and storage costs of fosphenytoin sodium injection. The structures of main degradation impurities generated during storage of fosphenytoin sodium are shown in Table 1 below.

**Table 1 Structures of degradation impurities of fosphenytoin sodium**

| Names of Impurities | Structures of Impurities | Abbreviations of Impurities |
|---|---|---|
| Diphenylglycine | 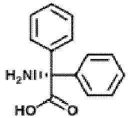 | Impurity A |
| Diphenyl hydantoic acid | 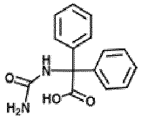 | Impurity B |
| Phenytoin | 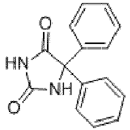 | Impurity C |

In view of this, it is urgent to develop an alternative dosage form of fosphenytoin sodium, so as to solve the above-mentioned technical problems.

U.S. patent document No.4925860 discloses a pharmaceutical composition of fosphenytoin sodium. It is described in this patent document that fosphenytoin sodium is prone to degradation, and the degradation products include formaldehyde, 5,5-diphenyl-4-imidazolidinone (DIZ), diphenylglycinamide, benzophenone, Impurity A and Impurity C. It is indicated in this patent that formation rate of phenytoin increases as the pH is lowered, and the solubility of Impurity C decreases at a lower pH. Since Impurity C is water-insoluble, it precipitates in an aqueous preparation of fosphenytoin sodium, thereby diminishing the shelf life of fosphenytoin sodium and causing problem of visible particulate matter. This patent document teaches that a suitable organic buffer such as tromethamine can be used to maintain a pH range from 8.3 to 9.4, which allows the degradation product of fosphenytoin sodium to be primarily diphenylglycinamide and minimizes generation of Impurity C, thereby extending the shelf life of fosphenytoin sodium. The pH range of the composition meets requirements of United States Pharmacopeia (USP 28). However, it is not taught in this patent as for how to avoid degradation of fosphenytoin sodium, or how to obtain a fosphenytoin sodium preparation that can be stored at room temperature.

Inventors of the present disclosure have found that pharmaceutical composition prepared according to the formula provided in the U.S. patent document No.4925860 is unstable if stored at 25°C and 40°C and Impurity B will be generated.

European patent document No. EP2303228B1 discloses fosphenytoin sodium liquid preparations comprising different buffers. The invention claims an aqueous pharmaceutical composition comprising fosphenytoin or a salt thereof, and a buffer selected from sodium bicarbonate, sodium phosphate, boric acid or glycine, wherein the composition has a pH of less than 8.3. The invention does not teach whether these compositions are stable, or whether these compositions can be lyophilized so as to obtain lyophilized compositions that are stable at room temperature.

The inventors of the present disclosure have found that the fosphenytoin sodium liquid preparations prepared using the formula disclosed in the patent document No. EP2303228B1 is unstable if stored at 25°C and 40°C and Impurity B will be generated. However, if these liquid preparations are lyophilized, they are still unstable if stored at 25°C and 40°C and Impurity A will be generated.

Patent document No. US6133248 describes a pharmaceutical composition with extended shelf life, comprising fosphenytoin sodium, cyclodextrin and a pharmaceutically acceptable carrier. This patent document shows that about 10 µg/mL of Impurity C will be newly generated if the pharmaceutical composition is placed at 37°C for 10 days. Impurity C is insoluble in aqueous conditions, and the cyclodextrin in this patent document plays the role of increasing the solubility of Impurity C without affecting the generation rate thereof. In addition, this patent document does not provide a method for effectively reducing the generation of impurity C and other impurities.

Patent document No. WO9904798A1 describes a lyophilized fosphenytoin sodium composition, which can be reconstituted with addition of a pharmaceutically acceptable diluent (preferably water). According to the afore-mentioned patent document, fosphenytoin sodium, which was formulated and lyophilized in 100 mM tromethamine buffer, reaches solubility greater than 140 mg/mL when reconstituted. However, the afore-mentioned patent does not teach whether the lyophilized preparation of fosphenytoin sodium is stable. The inventors of the present disclosure have found that, if the lyophilized fosphenytoin sodium composition comprising a buffer solution (including tromethamine) is placed at 25°C and 40°C for 14 days, Impurity A will be generated.

Conventional lyophilization process includes steps of:
1) preparation of product (pre-treatment);
2) freezing (pre-freezing) of the product: freezing the product into a solid state;
3) a first stage of drying (sublimation drying): removing ice crystals in the product by means of sublimation;
4) a second stage of drying (analytical drying): evaporating part of moisture remaining in the product at a higher temperature so that residual moisture meet predetermined requirements; and
5) sealing and packaging.

In the first stage of drying, materials need to absorb heat. If a drug is not heated or the heat is insufficient, moisture will absorb the heat of the drug itself during sublimation and thus reduces the temperature of the drug, which causes a vapor pressure of the drug to decrease, thereby decreasing the sublimation rate, prolonging the entire drying time and reducing the rate of production; if the drug is over-heated, the sublimation rate of the drug will be increased, but excessive heat will enhance the temperature of the frozen drug itself after offsetting the heat absorbed by the sublimation of the drug, which causes the drug to partially or even completely melt and results in a phenomenon of drying shrinkage and foaming of the drug, and the whole drying process may fail. Therefore, in the first stage of drying, a suitable amount of heat must be applied during the first stage of drying, and the freeze-dried layer must be controlled to be below the eutectic point of the product, so as to prevent the ice crystals from melting.

The second stage of drying is also called analytical drying. After the first stage of drying, there is still a part of moisture, which is not frozen, adsorbed on capillary walls and polar groups of the dried material. When the moisture reaches certain content, it provides conditions for the growth and reproduction of microorganisms, as well as certain chemical reactions. Experiments have shown that even a low moisture content adsorbed by a monolayer may result in a solution of certain compounds, as a result of which the same mobility and reactivity as an aqueous solution are produced. Therefore, in order to improve the storage stability of the product and extend its shelf life, it is necessary to remove the moisture as much as possible.

In conventional lyophilization process, it is selected to extend lyophilization time so as to better remove the moisture compriseed in the product; as a result, the total lyophilization time of the product is too long, resulting in an increase in the lyophilization time cost and a decrease in lyophilization efficiency. There is no research report on efficient lyophilization method of fosphenytoin sodium.

### Summary of the Invention

In view of the technical problems of fosphenytoin sodium preparations existing in the prior art, such as easy degradation and inability to be stored at room temperature, the present disclosure aims to provide a fosphenytoin sodium solid composition so as to solve the above-mentioned technical problems. Another object of the present disclosure is to provide an efficient fosphenytoin sodium lyophilization method.

The present disclosure provides a fosphenytoin sodium solid composition comprising fosphenytoin sodium and at least one carbohydrate.

Preferably, the solid composition can be stored at room temperature.

Preferably, the solid composition is a lyophilized composition.

Preferably, the carbohydrate is at least one selected from sugar and oligosaccharide.

Preferably, the sugar is at least one selected from monosaccharide, disaccharide and sugar alcohol.

Preferably, the monosaccharide is at least one selected from glucose, galactose and fructose.

Preferably, the disaccharide is at least one selected from sucrose, lactose, trehalose, maltose and isomaltose.

Preferably, the sugar alcohol is at least one selected from sorbitol, mannitol, xylitol and maltitol.

Preferably, the oligosaccharide is at least one selected from raffinose, stachyose, isomaltooligosaccharide, fructooligosaccharide, mannose oligosaccharide and soybean oligosaccharide.

Preferably, prior to lyophilization, the weight-to-volume ratio of the carbohydrate in the composition is 1% to 20%, preferably 3% to 15%, and more preferably 5% to 10%.

Preferably, the solid composition further comprises a buffer.

Preferably, the buffer is one or more selected from phosphate buffer, hydrophosphate buffer, dihydrogen phosphate buffer, bicarbonate buffer, carbonate buffer, boric acid buffer, borate buffer, amino acid buffer, trialkylamine buffer, tromethamine buffer, pyrophosphate buffer, Glycyl Glycine (glycylglycine) buffer; preferably, the phosphate, hydrophosphate, dihydrogen phosphate, bicarbonate, carbonate, borate and pyrophosphate are independently a sodium salt and/or a potassium salt; and the trialkylamine is trimethylamine.

Preferably, prior to lyophilization, the concentration of the buffer is 10 to 150 mM, and more preferably 20 to 100 mM.

Preferably, the pH of the fosphenytoin sodium solid composition prior to lyophilization or after reconstitution is 8 to 10, preferably 8 to 9.3, and more preferably 8 to 9.

Preferably, prior to lyophilization, the concentration of the fosphenytoin sodium is 75 mg/mL to 150 mg/mL, preferably 75 mg/mL to 100 mg/mL; and more preferably 75 mg/mL or 100 mg/mL.

Preferably, after reconstitution, the concentration of the fosphenytoin sodium is 75 mg/mL to 150 mg/mL, preferably 75 mg/mL to 100 mg/mL; and more preferably 75 mg/mL.

Preferably, no Impurity A, Impurity B or Impurity C is generated in the fosphenytoin sodium solid composition after being placed at 25°C and 60% relative humidity for 14 days.

Preferably, no Impurity A, Impurity B or Impurity C is generated in the fosphenytoin sodium solid composition after being placed at 25°C and 60% relative humidity for 3 months.

Preferably, no Impurity A, Impurity B or Impurity C is generated in the fosphenytoin sodium solid composition after being placed at 40°C and 75% relative humidity for 14 days.

Preferably, no Impurity A, Impurity B or Impurity C is generated in the fosphenytoin sodium solid composition after being placed at 40°C and 75% relative humidity for 3 months.

Preferably, the solid composition is a lyophilized composition comprising fosphenytoin sodium, a buffer and at least one carbohydrate; the buffer is tromethamine; the carbohydrate is selected from trehalose, sucrose, mannitol or lactose; and the pH of the lyophilized composition before lyophilization or after reconstitution is 8 to 9.

Preferably, prior to lyophilization, the concentration of the fosphenytoin sodium is 75 mg/mL or 100 mg/mL, the concentration of the buffer is 20 to 100 mM, and the weight-to-volume ratio of the carbohydrate in the composition is 5% to 10 %.

For another aspect, the present disclosure further provides a lyophilization method of fosphenytoin sodium, wherein the total lyophilization time of the lyophilization method is reduced by more than 60% relative to a total lyophilization time of conventional lyophilization methods, preferably reduced by more than 70%.

Preferably, the method includes: (1) preparing a fosphenytoin sodium solution; (2) pre-freezing the fosphenytoin sodium solution; and (3) directly increasing the shelf temperature at a certain heating rate to a predetermined temperature for drying, rather than individually setting a sublimation drying step and an analytical drying step.

Preferably, the fosphenytoin sodium solution comprises fosphenytoin sodium and at least one carbohydrate.

Preferably, the carbohydrate is at least one selected from sugar and oligosaccharide, and the sugar is at least one selected from monosaccharide, disaccharide and sugar alcohol.

Preferably, the monosaccharide is at least one selected from glucose, galactose and fructose.

Preferably, the disaccharide is at least one selected from sucrose, lactose, trehalose, maltose and isomaltose.

Preferably, the sugar alcohol is at least one selected from sorbitol, mannitol, xylitol and maltitol.

Preferably, the oligosaccharide is at least one selected from raffinose, stachyose, isomaltooligosaccharide, fructooligosaccharide, mannose oligosaccharide and soybean oligosaccharide.

Preferably, the weight-to-volume ratio of the carbohydrates is 1% to 20%, preferably 3% to 15%, and more preferably 5% to 10%.

Preferably, the pH value of the fosphenytoin sodium solution is 8 to 10, preferably 8 to 9.3, and more preferably 8 to 9.

Preferably, the fosphenytoin sodium solution further comprises a buffer.

Preferably, the buffer is one or more selected from phosphate buffer, hydrophosphate buffer, dihydrogen phosphate buffer, bicarbonate buffer, carbonate buffer, boric acid buffer, borate buffer, amino acid buffer, trialkylamine buffer, tromethamine buffer, pyrophosphate buffer, Glycyl Glycine (glycylglycine) buffer; preferably, the phosphate, hydrophosphate, dihydrogen phosphate, bicarbonate, carbonate, borate, pyrophosphate are independently a sodium salt and/or a potassium salt; and the trialkylamine is trimethylamine.

Preferably, the concentration of the buffer is 10 to 150 mM, and more preferably 20 to 100 mM.

Preferably, the concentration of the fosphenytoin sodium is 75 mg/mL to 150 mg/mL; more preferably 75 mg/mL to 100 mg/mL; and further preferably 75 mg/mL or 100 mg/mL.

Preferably, in the lyophilization method, the pre-freezing temperature in Step (2) is -40°C to -60°C, preferably -45°C to -55°C, and more preferably -45°C to -50°C.

Preferably, the cooling rate of decreasing the temperature to the pre-freezing temperature is 0.5 to 6 °C/min, preferably 1 to 5 °C/min, and more preferably 1 to 1.5 °C/min.

Preferably, the heating rate in Step (3) is 0.01 to 5 °C/min, preferably 0.025 to 3 °C/min, and more preferably 0.05 to 1.5 °C/min.

Preferably, the shelf temperature is directly increased to 5°C to 25°C, preferably to 10°C to 25°C, and more preferably to 20°C to 25°C.

Preferably, the fosphenytoin sodium solid composition is used for treatment of epilepsy or other convulsive states.

For another aspect, the present disclosure further provides use of the fosphenytoin sodium solid composition in preparation of a medicament for treatment of epilepsy or other convulsive states.

For another aspect, the present disclosure further provides a method for treatment of epilepsy or other convulsive states, including administering to a patient in need thereof an effective amount of the afore-mentioned fosphenytoin sodium solid composition for treatment of epilepsy or other convulsive states.

In the specification and claims of the present disclosure, unless otherwise specified, the scientific and technical terms used in the present disclosure have the meanings commonly understood by those skilled in the art. However, for better understanding of the present disclosure, definitions and explanations of some related terms are provided below.

Carbohydrate classification in the present disclosure is cited from the document "Carbohydrates in human nutrition: Report of a joint FAO/WHO expert consultation, Rome, 14-18 April 1997", wherein: sugar refers to a general term for sugars with a polymerization degree of 1 to 2, including monosaccharides, disaccharides and sugar alcohols, such as glucose, galactose, fructose, sucrose, lactose, trehalose, maltose, isomalt, sorbitol, mannitol, Xylitol, and Maltitol. Oligosaccharide refers to a general term for sugars with a polymerization degree of 3 to 9, such as raffinose, stachyose, isomaltooligosaccharide, fructooligosaccharide, mannose oligosaccharide and soybean oligosaccharide.

The percentage (%) of carbohydrates in the present disclosure refers to the weight-to-volume ratio, and the "weight-to-volume ratio" refers to the weight (with "g" as the unit) of the component in every 100 mL of the liquid system, i.e. g/100 mL.

The concentration unit mM of the buffer in the present disclosure refers to millimolar concentration, and the "millimolar concentration" refers to the number of millimolars (with "mmol" as the unit) of the component in every 1 L of the liquid system, i.e. mmol/L.

In all stability researches in the present disclosure, the relative humidity is 60% when at 25°C; and the relative humidity is 75% when at 40°C.

In the present disclosure, RP-HPLC refers to reversed-phase high performance liquid chromatography.

The detection limit in the present disclosure refers to the minimum amount of an analyte in the sample that can be detected, wherein, in the present discosure, the detection limit of Impurity A is 11.0 pg, the detection limit of Impurity B is 6.3 pg, and the detection limit of Impurity C is 4.5 ng.

In the present disclosure, "no impurity is generated", "no Impurity A, Impurity B, or Impurity C is generated", "no Impurity A, Impurity B or Impurity C is detected, and no other impurities are detected", "no Impurity A, Impurity B, Impurity C or other impurities is generated", "no impurity appears" and other similar expressions all mean that corresponding impurity in the analyte is lower than the detection limit.

In the present disclosure, the expression "Not detected" means that corresponding impurity in the analyte is lower than the detection limit.

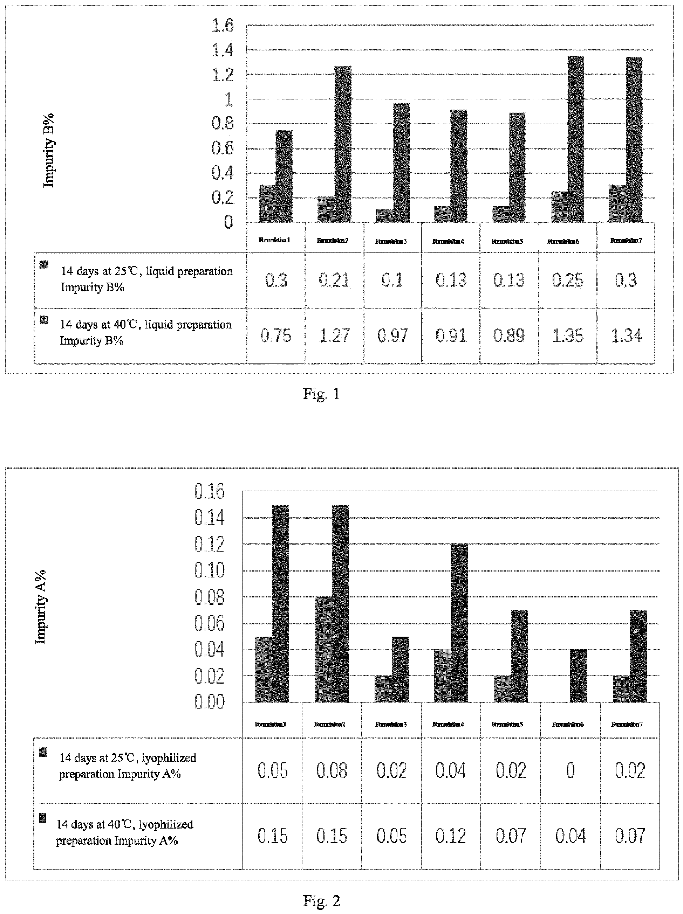

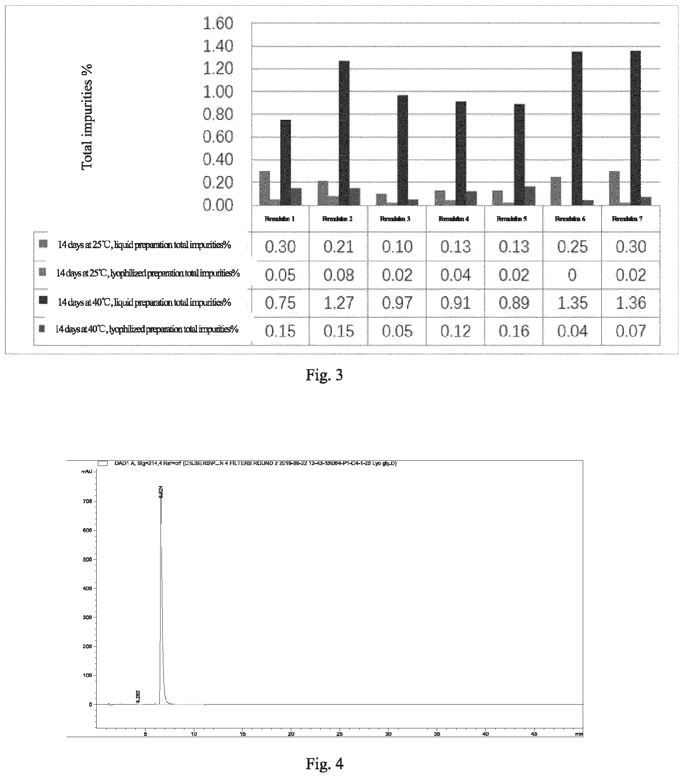

In the present disclosure, the impurity content in Fig. 2 and Fig. 3 is 0, indicating that corresponding impurity is not detected, namely, the impurity is lower than the detection limit.

"N/A" in the present invention means not applicable.

"PES" in the present disclosure means polyethersulfone.

Moisture content in the present disclosure is measured by Karl Fischer titration.

In the present disclosure, FTS Lyo Star II lyophilizer is adopted for lyophilization.

The term "reconstituted" in the present disclosure means that each component of a lyophilized preparation is dissolved with an aqueous solution (including but not limited to ethanol, water, buffer, sodium chloride solution, aqueous dextrose solution or mixtures thereof, such as water for injection, physiological saline, etc.), so as to obtain the reconstituted pharmaceutical preparation. The reconstitution of the lyophilized preparation in the present disclosure can be carried out by technical means generally known to those skilled in the art. Therefore, a reconstituted preparation can be obtained after reconstitution of the lyophilized preparation.

### Beneficial Effects

The fosphenytoin sodium solid composition provided by the invention is stable and can be stored at room temperature. The commercially available fosphenytoin sodium injection must be stored at 2°C to 8°C, and, even if it is stored at such a low temperature, impurities are still inevitable.

As one aspect of the present disclosure, a fosphenytoin sodium solid composition is provided which has been placed for 14 days at 25°C with a humidity of 60%, as well as at 40°C with a humidity of 75% while it is free of Impurity A, Impurity B and Impurity C.

As another aspect of the present disclosure, a fosphenytoin sodium solid composition is provided, which has been placed for 1 month, 2 months and 3 months at 2°C to 8°C and at 25°C with a humidity of 60%, as well as at 40°C with a humidity of 75% while it is free of Impurity A, Impurity B and Impurity C.

In addition, an unconventional fosphenytoin sodium lyophilization method is provided in the present disclosure, which needs no independent sublimation drying stage or analytical drying stage, instead, the shelf temperature is directly increased at a certain heating rate to a predetermined temperature (such as 20°C, etc.) for lyophilization, the moisture content meets the quality requirements, the lyophilization time is significantly shortened, and the lyophilized product is excellent in reconstitutability and can be completely reconstituted within 1 minute, which is convenient for clinical use.

As another aspect of the present disclosure, a lyophilization process is provided, wherein the lyophilized product is stable in quality, and can be placed for 14 days at 25°C with a humidity of 60%, as well as at 40°C with a humidity of 75% while it is free of Impurity A, Impurity B and Impurity C.

As another aspect of the present disclosure, a lyophilization process is provided, wherein the lyophilized product is stable in quality, and can be placed for 1 month, 2 months and 3 months at 2°C to 8°C and at 25°C with a humidity of 60%, as well as at 40°C with a humidity of 75% while it is free of Impurity A, Impurity B and Impurity C.

### Description of Drawings

Fig. 1: Histogram of the percentage content of Impurity B after the liquid preparation in Experiment 1 has been placed for 14 days at 25°C and at 40°C, wherein: the histogram on the left corresponding to each sample is the sample data graph after being placed for 14 days at 25°C, and the histogram on the right is the sample data graph after being placed for 14 days at 40°C.
Fig. 2: Histogram of the percentage content of Impurity A after the lyophilized preparation in Experiment 1 has been placed for 14 days at 25°C and at 40°C, wherein: the histogram on the left corresponding to each sample is the sample data graph after being placed for 14 days at 25°C, and the histogram on the right is the sample data graph after being placed for 14 days at 40°C.
Fig. 3: Histogram of the percentage content of total impurities in the liquid preparation and lyophilized preparation in Experiment 1 after being placed for 14 days at 25°C and at 40°C.
Fig. 4: Chromatogram of the lyophilized preparation comprising glycine in Experiment 1 after being placed for 14 days at 40°C.
Fig. 5: Amplified chromatogram of the lyophilized preparation comprising glycine in Experiment 1 after being placed for 14 days at 40°C.
Fig. 6: Lyophilization pressure curve (with a filling volume of 1.5 mL) for lyophilization using scaled-up lyophilization process #7-1 in Experiment 5; wherein: the line CM represents the pressure setting value of a lyophilizer; and the line Pirani represents the pressure monitoring values of the lyophilizer.
Fig. 7: Lyophilization temperature curve (with a filling volume of 1.5 mL) for lyophilization using scaled-up lyophilization process #7-1 in Experiment 5; wherein: the line SHELF SETPT represents the shelf temperature setting value; the line Left represents the temperature monitoring value of a probe on the left side in the lyophilizer; and the line Right represents the temperature monitoring value of a probe on the right side in the lyophilizer.
Fig. 8: Lyophilization pressure curve (with a filling volume of 7.5 mL) for lyophilization using scaled-up lyophilization process #7-2 in Experiment 5; wherein: the line CM represents the pressure setting value of a lyophilizer; and the line Pirani represents the pressure monitoring values of the lyophilizer.
Fig. 9: Lyophilization temperature curve (with a filling volume of 7.5 mL) for lyophilization using scaled-up lyophilization process #7-2 in Experiment 5; wherein: the line SHELF SETPT represents the shelf temperature setting value the line Left represents the temperature monitoring value of a probe on the left side in the lyophilizer; and the line Right represents the temperature monitoring value of a probe on the right side in the lyophilizer.
Fig. 10: Appearance diagram of a representative sample of the lyophilized preparation obtained by lyophilization using the scaled-up lyophilization process #7-1 in Experiment 5 (with a filling volume of 1.5 mL).
Fig. 11: Bottom-of-the-bottle view of a representative sample of the lyophilized preparation obtained by lyophilization using the scaled-up lyophilization process #7-1 in Experiment 5 (with a filling volume of 1.5 mL).
Fig. 12: Appearance diagram of a representative sample of the lyophilized preparation obtained by lyophilization using the scaled-up lyophilization process #7-2 in Experiment 5 (with a filling volume of 7.5 mL).
Fig. 13: Bottom-of-the-bottle view of a representative sample of the lyophilized preparation obtained by lyophilization using the scaled-up lyophilization process #7-2 in Experiment 5 (with a filling volume of 7.5 mL).

### Embodiments

Embodiments of the present disclosure are described in detail via following examples, which are only used to illustrate the present disclosure, rather than limiting the scope of the present disclosure.

### Materials and methods

### Analysis and detection method

The analysis and detection method used in the present disclosure is the same as that of fosphenytoin sodium injection in the United States Pharmacopoeia (USP 42). The specific detection method is the reversed-phase high-performance chromatography, and the detection conditions are as follows:

### Chromatographic conditions

Instrument: high-performance liquid chromatograph Agilent 1290 Infinity;
Chromatographic column: ZORBAX Eclipse SB 80A Phenyl, 4.6 × 150mm, 3.5 µm;
Mobile phase: methanol:acetonitrile:buffer (pH of 8.2 g/L potassium dihydrogen phosphate solution was adjusted to 6.5 with 6 mol/L potassium hydroxide solution)= 25:2:73;
Elution mode: isocratic elution;
Detection wavelength: 214 nm;
Concentration of sample to be tested: 0.15 mg/mL;
Flow rate: 1.25 mL/min;
Sample size: 40 µL;
System applicability: Impurity A, Impurity B, Impurity C;
Separation degree: the separation degree between Impurity A and Impurity B is not less than 4.0;
Tailing factor: the tailing factor of fosphenytoin sodium peak is not greater than 1.8;
%RSD: a relative standard deviation of the peak area of fosphenytoin sodium is not more than 1.0%;

Number of theoretical plates: not less than 2,250 if calculated according to the peak of fosphenytoin sodium.

### (2) Solution preparation:

Buffer preparation: 8.2 g/L potassium dihydrogen phosphate aqueous solution was prepared and pH thereof was adjusted to 6.5±0.05 with 6 N potassium hydroxide solution.

Reference substance stock solution A: a suitable amount of fosphenytoin sodium reference substance was taken, and was dissolved with a small amount of methanol, and then was diluted with mobile phase to make a solution comprising about 0.75 mg of fosphenytoin sodium in each 1 mL.

Reference substance stock solution B: a suitable amount of diphenylglycine (Impurity A) reference substance, a suitable amount of diphenyl hydantoic acid (Impurity B) reference substance and a suitable amount of phenytoin reference substance (Impurity C) were dissolved in methanol and were quantitatively diluted, thereby preparing a solution comprising about 7.5 µg of Impurity A, about 15 µg of Impurity B and about 7.5 µg of Impurity C in each 1 mL.

Reference substance solution: a suitable amount of reference substance stock solution A and a suitable amount of reference substance stock solution B were quantitatively diluted with buffer, thereby preparing a solution comprising about 150 µg of fosphenytoin sodium, about 0.75 µg of Impurity A, about 1.5 µg of Impurity B and about 0.75 µg of Impurity C in each 1 mL.

Test solution (liquid preparation): 7.5 µL of 100 mg/mL fosphenytoin sodium liquid preparation was taken, and was diluted to 5 mL with a buffer solution (pH 6.5) comprising 10% methanol, thereby preparing a solution comprising about 150 µg of fosphenytoin sodium in each 1 mL.

Test solution (lyophilized preparation): 10 µL of 75 mg/mL reconstituted solution of fosphenytoin sodium lyophilized preparation was taken, and was diluted to 5 mL with a buffer solution (pH 6.5) comprising 10% methanol, thereby preparing a solution comprising about 150 µg of fosphenytoin sodium in each 1 mL.

### pH

The pH of the fosphenytoin sodium preparation samples was measured by means of a Thermo Scientific, OrionStar Model A 211 pH meter equipped with a Ross PerpHecT microelectrode (with a model number of 8220BNWP). For buffer solution preparations, pH was measured by means of triode electrodes (Thermo Scientific, US Gel-filled Ultra Triode Electrodes). The instrument was normalized with pH 4, 7 and 10 buffers prior to each use.

### Karl-Fischer

Moisture content was measured by means of a Mettler Toledo DL36 KF Coulometer and a Mettler Toledo DO305 Drying Oven. The instrument was calibrated against KF-Oven (Sigma, 34784, Lot# SZBD 226AV) with Hydranal water standards. Approximately 50 mg of lyophilized powder was transferred to a vial of 3 mL and 13 mm and the vial was then stoppered. The vial with the lyophilized powder was heated at 100°C in a drying oven, and residual water vapor in the sample was bubbled into a Hydranal (Sigma, 34836, Lot#SZBE 2830V) container with cathode and anode solutions, so as to titrate the produced water vapour by using the Coulometric method.

### Experiment 1 Research on stability of 75 mg/mL fosphenytoin sodium preparations comprising different buffers

The inventors placed 75 mg/mL fosphenytoin sodium liquid preparations and lyophilized preparations comprising different or no buffers for 14 days at 25°C and at 40°C, and evaluated stability thereof.

**Table 2 Formulations of fosphenytoin sodium solutions before lyophilization, the solutions having a concentration of 75 mg/mL and comprising different or no buffers**

| No. | Formulation Composition | | | | Solution pH |
|---|---|---|---|---|---|
| | Active Components | Buffers | Solvents | pH Regulator | |
| Formulation 1 | 75 mg/mL fosphenytoin sodium | / | water for injection | 1N HCl or NaOH solution | 8.54 |
| Formulation 2 | 75 mg/mL fosphenytoin sodium | 100 mM sodium pyrophosphate | water for injection | 1N HCl or NaOH solution | 8.63 |
| Formulation 3 | 75 mg/mL fosphenytoin sodium | 100 mM sodium phosphate | water for injection | 1N HCl or NaOH solution | 8.55 |
| Formulation 4 | 75 mg/mL fosphenytoin | 100 mM glycine | water for injection | 1N HCl or NaOH solution | 8.99 |
| | sodium | | | | |
| Formulation 5 | 75 mg/mL fosphenytoin sodium | 100 mM glycylglycine | water for injection | 1N HCl or NaOH solution | 8.87 |
| Formulation 6 | 75 mg/mL fosphenytoin sodium | 100 mM sodium bicarbonate | water for injection | 1N HCl or NaOH solution | 9.00 |
| Formulation 7 | 75 mg/mL fosphenytoin sodium | 100 mM sodium borate | water for injection | 1N HCl or NaOH solution | 8.94 |

| | | | | | |
|---|---|---|---|---|---|
| Note: "/" indicates that there is no such component in the formulation. | | | | | |

### Preparation method:

(1) a prescribed amount of buffer was added into water for injection, the pH was adjusted to 8.8 with a pH regulator, and water for injection was added to a constant volume of 100 mL, as a result, a buffer solution with a concentration of 100 mM was prepared, and was then filtered with 0.2 µm PES filter membrane; and
(2) the buffer solution obtained in step (1) was adopted, and fosphenytoin sodium was added to prepare a fosphenytoin sodium liquid preparation with a concentration of 75 mg/mL, the liquid preparation was filled into a 5 mL vial at a 2 mL specification, and was lyophilized by means of a lyophilization process, as a result, a fosphenytoin sodium lyophilized preparation was prepared. The lyophilization parameters of the lyophilized preparations are shown in Table 3.

**Table 3 Lyophilization parameters of lyophilized preparations**

| Steps | Shelf Temperature | Heating Rate (°C/min) | Holding Time (h) | Pressure |
|---|---|---|---|---|
| Loading samples | room temperature | N/A | N/A | N/A |
| Pre-freezing | -50°C | -1 °C/min | 2 | N/A |
| Sublimiation drying | -30°C | 1 °C/min | 32.3 | 70 mTorr |
| Analytical drying | 20°C | 1 °C/min | 7.0 | 70 mTorr |

Table 4 lists the RP-HPLC analysis results of concentrations and contents of fosphenytoin sodium liquid preparations before lyophilization and on the 0^{th} day after reconstitution of lyophilized preparations, the preparations comprising different buffers. The data show that the concentrations of the preparations before and after lyophilization were within the target range. According to the United States Pharmacopeia (USP-42), the acceptable content limit for fosphenytoin sodium is 90% to 110%. The preparation (Formulation 5) comprising glycylglycine has a slightly higher content of 111% to 112%.

**Table 4**

| No. | The 0^{th} day | | | |
|---|---|---|---|---|
| | Liquid Preparations | | Lyophilized Preparations | |
| | Concentration (mg/mL) | Content % | Concentration (mg/mL) | Content % |
| Formulation 1 | 79.2 | 105.6 | 76.7 | 102.3 |
| Formulation 2 | 76.1 | 101.5 | 73.1 | 97.4 |
| Formulation 3 | 77.1 | 102.9 | 76.2 | 101.6 |
| Formulation 4 | 79.6 | 106.2 | 78.1 | 104.1 |
| Formulation 5 | 83.4 | 111.2 | 84.7 | 112.9 |
| Formulation 6 | 78.1 | 104.1 | 82.6 | 110.2 |
| Formulation 7 | 81.7 | 108.9 | 77.9 | 103.9 |

Tables 5 and 6 list concentrations and contents of liquid preparations and lyophilized preparations after the preparations had been placed for 14 days at 25°C and at 40°C. The data indicate that the liquid preparations and lyophilized preparations were essentially at the target concentration.

**Table 5**

| No. | Placed for 14 days at 25°C | | | |
|---|---|---|---|---|
| | Liquid Preparations | | Lyophilized Preparations | |
| | Concentration (mg/mL) | Content % | Concentration (mg/mL) | Content % |
| Formulation 1 | 70.8 | 94.4 | 83.0 | 110.7 |
| Formulation 2 | 85.2 | 113.6 | 74.6 | 99.5 |
| Formulation 3 | 69.4 | 92.5 | 76.8 | 102.4 |
| Formulation 4 | 75.2 | 100.2 | 77.2 | 102.9 |
| Formulation 5 | 68.3 | 91.1 | 78.7 | 104.9 |
| Formulation 6 | 78.2 | 104.2 | 61.6 | 82.1 |
| Formulation 7 | 96.8 | 129.1 | 79.1 | 105.5 |

**Table 6**

| No. | Placed for 14 days at 40°C | | | |
|---|---|---|---|---|
| | Liquid Preparations | | Lyophilized Preparations | |
| | Concentration (mg/mL) | Content % | Concentration (mg/mL) | Content % |
| Formulation 1 | 70.2 | 93.6 | 76.3 | 101.8 |
| Formulation 2 | 74.4 | 99.2 | 80.1 | 106.8 |
| Formulation 3 | 71.7 | 95.6 | 76.0 | 101.4 |
| Formulation 4 | 79.7 | 106.3 | 77.3 | 103.0 |
| Formulation 5 | 67.5 | 90.0 | 79.0 | 105.4 |
| Formulation 6 | 76.1 | 101.4 | 77.6 | 103.5 |
| Formulation 7 | 77.4 | 103.2 | 76.0 | 101.3 |

Tables 7 and 8 list the changes in purity after fosphenytoin sodium preparations comprising different buffers had been placed for 14 days before and after lyophilization.

**Table 7**

| No. | Liquid Preparations | | | | | | |
|---|---|---|---|---|---|---|---|
| | The 0^{th} day | Placed for 14 days at 25°C | | | Placed for 14 days at 40°C | | |
| | Purity % | Impurity A % | Impurity B % | Impurity C % | Impurity A % | Impurity B % | Impurity C % |
| Formulation 1 | 100 | Not detected | 0.30 | Not detected | Not detected | 0.75 | Not detected |
| Formulation 2 | 100 | Not detected | 0.21 | Not detected | Not detected | 1.27 | Not detected |
| Formulation 3 | 100 | Not detected | 0.10 | Not detected | Not detected | 0.97 | Not detected |
| Formulation 4 | 100 | Not detected | 0.13 | Not detected | Not detected | 0.91 | Not detected |
| Formulation 5 | 100 | Not detected | 0.13 | Not detected | Not detected | 0.89 | Not detected |
| Formulation 6 | 100 | Not detected | 0.25 | Not detected | Not detected | 1.35 | Not detected |
| Formulation 7 | 100 | Not detected | 0.30 | Not detected | 0.02 | 1.34 | Not detected |

**Table 8**

| No. | Lyophilized Preparations | | | | | | |
|---|---|---|---|---|---|---|---|
| | The 0^{th} day | Placed for 14 days at 25°C | | | Placed for 14 days at 40°C | | |
| | Purity % | Impurity A % | Impurity B % | Impurity C % | Impurity A % | Impurity B % | Impurity C % |
| Formulation 1 | 100 | 0.05 | Not detected | Not detected | 0.15 | Not detected | Not detected |
| Formulation 2 | 100 | 0.08 | Not detected | Not detected | 0.15 | Not detected | Not detected |
| Formulation 3 | 100 | 0.02 | Not detected | Not detected | 0.05 | Not detected | Not detected |
| Formulation 4 | 100 | 0.04 | Not detected | Not detected | 0.12 | Not detected | Not detected |
| Formulation 5 | 100 | 0.02 | Not detected | Not detected | 0.07 | 0.09 | Not detected |
| Formulation 6 | 100 | Not detected | Not detected | Not detected | 0.04 | Not detected | Not detected |
| Formulation 7 | 100 | 0.02 | Not detected | Not detected | 0.07 | Not detected | Not detected |

As shown in Table 7 and Fig. 1, after liquid preparations had been placed for 14 days at 25°C and at 40°C, Impurity B was generated.

As shown in Table 8 and Fig. 2, after lyophilized preparations had been placed for 14 days at 25°C and at 40°C, Impurity A was generated.

Fig. 3 shows the percentage content of total impurities after the liquid preparations and the lyophilized preparations had been placed for 14 days at 25°C and at 40°C.

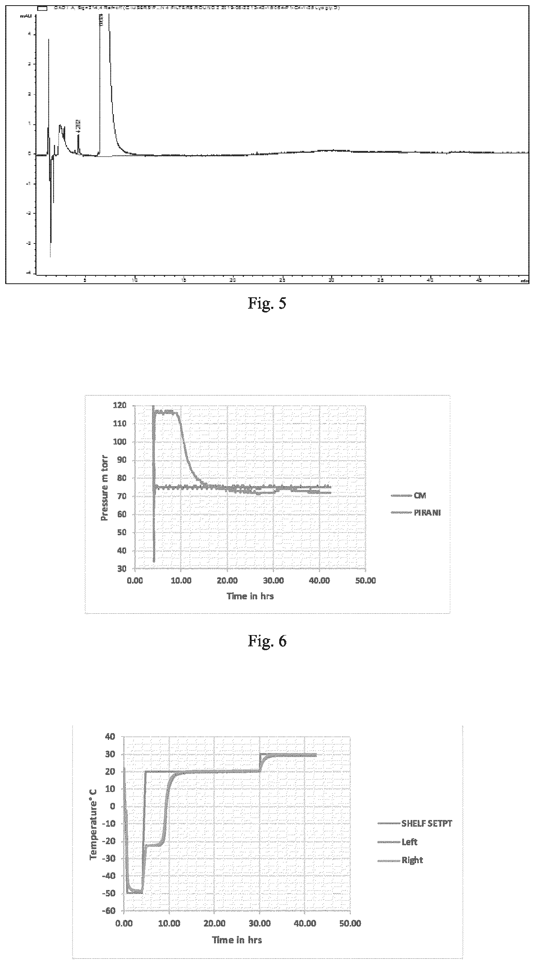

Figs. 4 and 5 are chromatograms of a representative lyophilized preparation (a lyophilized preparation comprising glycine), showing that Impurity A was generated after the representative lyophilized preparation had been placed for 14 days at 40°C.

### Experiment 2 Research on the effects of carbohydrates on the stability of fosphenytoin sodium preparations

Experiment 2 explores the effects on the stability of fosphenytoin sodium preparations in the presence of carbohydrates. The formulations are shown in Table 9.

**Table 9 Formulations of fosphenytoin sodium solution before lyophilization**

| No. | Formulation Composition | | | | | Solution pH |
|---|---|---|---|---|---|---|
| | Active Components | Buffers | Carbohydrates | Solvents | pH Regulators | |
| Formulation 8 | 75 mg/mL fosphenytoin sodium | / | / | water for injection | 1N HCl or NaOH solution | 8.87 |
| Formulation 9 | 75 mg/mL fosphenytoin sodium | / | 5% mannitol | water for injection | 1N HCl or NaOH solution | 8.77 |
| Formulation 10 | 75 mg/mL fosphenytoin sodium | 100 mM tromethamine | / | water for injection | 1N HCl or NaOH solution | 8.86 |
| Formulation 11 | 75 mg/mL fosphenytoin sodium | 100 mM tromethamine | 5% mannitol | water for injection | 1N HCl or NaOH solution | 8.90 |
| Formulation 12 | 75 mg/mL fosphenytoin sodium | 20 mM sodium carbonate | / | water for injection | 1N HCl or NaOH solution | 8.76 |
| Formulation 13 | 75 mg/mL fosphenytoin sodium | 20 mM sodium carbonate | 5% mannitol | water for injection | 1N HCl or NaOH solution | 8.88 |
| Formulation 14 | 75 mg/mL fosphenytoin sodium | 100 mM lysine | / | water for injection | 1N HCl or NaOH solution | 8.88 |
| Formulation 15 | 75 mg/mL fosphenytoin sodium | 100 mM lysine | 5% mannitol | water for injection | 1N HCl or NaOH solution | 8.86 |
| Formulation 16 | 75 mg/mL fosphenytoin sodium | 100 mM arginine | / | water for injection | 1N HCl or NaOH solution | 8.88 |
| Formulation 17 | 75 mg/mL fosphenytoin sodium | 100 mM arginine | 5% mannitol | water for injection | 1N HCl or NaOH solution | 8.768.88? |
| Formulation 18 | 75 mg/mL fosphenytoin sodium | 100 mM glycine | / | water for injection | 1N HCl or NaOH solution | 8.84 |
| Formulation 19 | 75 mg/mL fosphenytoin sodium | 100 mM tromethamine | 10% sucrose | water for injection | 1N HCl or NaOH solution | 8.88 |
| Formulation 20 | 75 mg/mL fosphenytoin sodium | 100 mM tromethamine | 10% trehalose | water for injection | 1N HCl or NaOH solution | 8.86 |
| Formulation 21 | 75 mg/mL fosphenytoin sodium | 100 mM tromethamine | 10% lactose | water for injection | 1N HCl or NaOH solution | 8.86 |
| Formulation 22 | 75 mg/mL fosphenytoin sodium | 10 mM sodium carbonate, 20 mM tromethamine | 5% mannitol | water for injection | 1N HCl or NaOH solution | 8.79 |

| | | | | | | |
|---|---|---|---|---|---|---|
| Note: "/" indicates that there is no such component in the formulation. | | | | | | |

Preparation method:
fosphenytoin sodium, buffers and carbohydrates in the above-mentioned formulations were added to water for injection; after stirring and dissolving, the pH was adjusted to 8.8±0.1 with a pH regulator; water for injection was added to a constant volume of 100 mL, and filtering was performed with 0.2 µm PES membrane, as a result, fosphenytoin sodium liquid preparations with concentrations shown in the formulations were prepared; liquid preparation was filled into a 3 mL vial at a 1 mL specification, and lyophilization preparation was performed according to the process parameters in Table 10.

**Table 10**

| Steps | Shelf Temperature | Heating Rate | Holding Time | Pressure |
|---|---|---|---|---|
| Loading | ambient temperature | N/A | N/A | N/A |
| Pre-freezing | -50°C | -1 °C/min | 2 h | N/A |
| Sublimiation drying | -30°C | 1 °C/min | 33 h | 70 mTorr |
| Analytical drying | 20°C | 1 °C/min | 7.1 h | 70 mTorr |

As shown by the experimental results, the concentrations of each liquid preparation and lyophilized preparation were basically target concentrations on the 0^{th} day and the 14^{th} day.

The RP-HPLC results of each preparation indicate that the RP-HPLC analytical purities of all liquid preparations and lyophilized preparations were 100% on the 0^{th} day.

Table 11 shows impurities of the liquid preparations and the lyophilized preparations of fosphenytoin sodium after being placed for 14 days respectively at 25°C and at 40°C.

**Table 11**

| Formulations | Liquid Preparations | | | | | | Lyophilized Preparations | | | | | |
|---|---|---|---|---|---|---|---|---|---|---|---|---|
| | Placed for 14 days at 25°C | | | Placed for 14 days at 40°C | | | Placed for 14 days at 25°C | | | Placed for 14 days at 40°C | | |
| | Impurity A % | Impurity B % | Impurity C % | Impurity A % | Impurity B % | Impurity C % | Impurity A % | Impurity B % | Impurity C % | Impurity A % | Impurity B % | Impurity C % |
| Formulation 8 | Not detected | 0.0944 | Not detected | Not detected | 0.5101 | Not detected | 0.0727 | Not detected | Not detected | 0.1583 | Not detected | Not detected |
| Formulation 9 | Not detected | 0.0769 | Not detected | Not detected | 0.4475 | Not detected | Not detected | Not detected | Not detected | Not detected | Not detected | Not detected |
| Formulation 10 | Not detected | 0.1472 | Not detected | Not detected | 0.5421 | Not detected | Not detected | Not detected | Not detected | 0.0448 | Not detected | Not detected |
| Formulation 11 | Not detected | 0.1465 | Not detected | Not detected | 0.5335 | Not detected | Not detected | Not detected | Not detected | Not detected | Not detected | Not detected |
| Formulation 12 | Not detected | 0.1524 | Not detected | Not detected | 0.9002 | Not detected | 0.0336 | Not detected | Not detected | 0.0850 | Not detected | 0.3225 |
| Formulation 13 | Not detected | 0.1747 | Not detected | Not detected | 1.0638 | Not detected | Not detected | Not detected | Not detected | Not detected | Not detected | Not detected |
| Formulation 14 | Not detected | 0.1832 | Not detected | Not detected | 0.6690 | Not detected | 0.0257 | Not detected | Not detected | 0.0590 | Not detected | 0.2048 |
| Formulation 15 | Not detected | 0.1696 | Not detected | Not detected | 0.6151 | Not detected | Not detected | Not detected | Not detected | Not detected | Not detected | Not detected |
| Formulation 16 | Not detected | 0.1483 | Not detected | Not detected | 0.5482 | Not detected | 0.0198 | Not detected | Not detected | 0.0649 | Not detected | Not detected |
| Formulation 17 | Not detected | 0.1346 | Not detected | Not detected | 0.5120 | Not detected | Not detected | Not detected | Not detected | Not detected | Not detected | Not detected |
| Formulation 18 | Not detected | 0.1307 | Not detected | Not detected | 0.5071 | Not detected | 0.0587 | Not detected | Not detected | 0.1349 | Not detected | 0.3153 |
| Formulation 19 | Not detected | 0.1321 | Not detected | Not detected | 0.4992 | Not detected | Not detected | Not detected | Not detected | Not detected | Not detected | Not detected |
| Formulation 20 | Not detected | 0.1260 | Not detected | Not detected | 0.4770 | Not detected | Not detected | Not detected | Not detected | Not detected | Not detected | Not detected |
| Formulation 21 | Not detected | 0.1187 | Not detected | Not detected | 0.3702 | Not detected | Not detected | Not detected | Not detected | Not detected | Not detected | Not detected |
| Formulation 22 | Not detected | 0.1403 | Not detected | Not detected | 0.6225 | Not detected | Not detected | Not detected | Not detected | Not detected | Not detected | Not detected |

As for a commercially available fosphenytoin sodium liquid preparation comprising 100 mM tromethamine and 75 mg/mL fosphenytoin sodium, it is disclosed in the specification that the pH range thereof is from 8.6 to 9.0, and the storage condition for this preparation is 2°C to 8°C. The present disclosure prepared a fosphenytoin sodium liquid preparation (Formulation 10) according to the commercially available formulation, and investigated its stability. As shown in Table 11, Impurity B of higher content was generated due to hydrolysis when the preparation was stored at 25°C and at 40°C. On the other hand, Impurity A was generated when the liquid preparation had been placed for 14 days at 40°C after lyophilization.

In addition, Impurity A was generated in all of the 75 mg/mL lyophilized preparations (Formulations 8, 10, 12, 14, 16, 18) of fosphenytoin sodium comprising water, sodium carbonate, lysine, arginine or glycine after the lyophilized preparations had been placed for 14 days at 40°C. Impurity C was generated in the fosphenytoin sodium lyophilized preparations (Formulations 12, 14, 18) comprising sodium carbonate, lysine or glycine.

Unexpectedly, in the presence of 5% lactose, 5% mannitol, 10% trehalose or 10% sucrose, no Impurity A, Impurity B and Impurity C, or other impurities were detected in all lyophilized preparations (Formulations 9, 11, 13, 15, 17, 19, 20, 21, 22) after they had been placed for 14 days at 25°C and at 40°C.

Apparently, in the presence of carbohydrates, 75 mg/mL fosphenytoin sodium lyophilized preparations respectively comprising water, tromethamine, sodium carbonate, arginine, lysine or glycine had no impurities generated after being placed for 14 days at 25°C and at 40°C. It indicates that carbohydrates in the lyophilized preparations can stabilize fosphenytoin sodium and prevent Impurity A and various other impurities from being generated by degradation.

Relative to liquid preparations, the lyophilized preparation disclosed in the present disclosure, after being placed at 25°C and 40°C, produces no impurities and is stable.

### Experiment 3 Research on stability of 100 mg/mL fosphenytoin sodium lyophilized preparations comprising carbohydrates

Experiment 2 shows that 75 mg/mL fosphenytoin sodium lyophilized preparations comprising carbohydrates had no impurities generated after being placed for 14 days at 40°C.

Experiment 3 explores stability of 100 mg/mL fosphenytoin sodium lyophilized preparations comprising carbohydrates (with or without tromethamine).

Generally, the loading quantity is one of the important parameters for lyophilization, especially the height of the filling volume. Therefore, it was explored to formulate API at greater concentrations so as to minimize the loading quantity of the 500 mg dose. Although formulation is performed at higher concentrations, effective dose remains the same or the formation can be performed at desired dose.

When the prepation concentration of fosphenytoin sodium was 112.5 mg/mL, clarity of the preparation comprising mannitol was better than that comprising trehalose or sucrose, indicating that the solubility of fosphenytoin sodium in mannitol solution is better and can be completely dissolved when diluted to 100 mg/mL.

The formulations of 100 mg/mL fosphenytoin solution before lyophilization and comprising carbohydrates, with or without buffers, are shown in Table 12.

**Table 12**

| No. | Formulation Composition | | | | | Solution pH |
|---|---|---|---|---|---|---|
| | Active Components | Buffers | Carbohydrates | Solvents | pH Regulator | |
| Formulation 23 | 100 mg/mL fosphenytoin sodium | / | 10% trehalose | water for injection | 1N HCl or NaOH solution | 8.88 |
| Formulation 24 | 100 mg/mL fosphenytoin sodium | 100 mM tromethamine | 10% trehalose | water for injection | 1N HCl or NaOH solution | 8.94 |
| Formulation 25 | 100 mg/mL fosphenytoin sodium | / | 10% sucrose | water for injection | 1N HCl or NaOH solution | 8.87 |
| Formulation 26 | 100 mg/mL fosphenytoin sodium | 100 mM tromethamine | 10% sucrose | water for injection | 1N HCl or NaOH solution | 8.95 |
| Formulation 27 | 100 mg/mL fosphenytoin sodium | / | 5% mannitol | water for injection | 1N HCl or NaOH solution | 8.87 |
| Formulation 28 | 100 mg/mL fosphenytoin sodium | 100 mM tromethamine | 5% mannitol | water for injection | 1N HCl or NaOH solution | 8.93 |

Preparation method:
carbohydrates, fosphenytoin sodium, and buffers in the above-mentioned formulations were added to water for injection; after stirring and dissolving, the pH was adjusted to 8.8±0.1 with a pH regulator; water for injection was added to a constant volume of 100 mL, and filtering was performed with 0.2 µm PES membrane, as a result, fosphenytoin sodium liquid preparations with concentrations shown in the formulations were prepared; the liquid preparations were filled into a 3 mL vial at a 1 mL specification, and lyophilization preparation was performed according to the process parameters in Table 13.

**Table 13**

| Steps | Shelf Temperature | Heating Rate | Holding Time | Pressure |
|---|---|---|---|---|
| Loading | ambient temperature | N/A | N/A | N/A |
| Pre-freezing | -50°C | -1 °C/min | 2 | N/A |
| Sublimiation drying | -30°C | 1 °C/min | 30.45 | 70 mTorr |
| Analytical drying | 20°C | 1 °C/min | 10 | 70 mTorr |

According to the test results of clarity, all liquid preparations were clear on the 0^{th} day and after being placed for 14 days at 40°C, and reconstituted preparations of all lyophilized preparations were clear on the 0^{th} day and after being placed for 14 days at 40°C.

Table 14 lists the measured concentrations of each liquid preparation and lyophilized preparation comprising 100 mg/mL fosphenytoin sodium. The concentration of each liquid preparation and lyophilized preparation was substantially at the target concentration after being placed for 14 days at 40°C.

**Table 14**

| No. | Placed for 14 days at 40°C | | | |
|---|---|---|---|---|
| | Liquid Preparations | | Lyophilized Preparations | |
| | Concentration (mg/ml) | Content % | Concentration (mg/ml) | Content % |
| Formulation 23 | 97.1 | 97.1 | 99.9 | 99.9 |
| Formulation 24 | 96.6 | 96.6 | 99.3 | 99.3 |
| Formulation 25 | 99.6 | 99.6 | 104.9 | 104.9 |
| Formulation 26 | 100.1 | 100.1 | 102.4 | 102.4 |
| Formulation 27 | 98.4 | 98.4 | 98.2 | 98.2 |
| Formulation 28 | 101.0 | 101.0 | 104.5 | 104.5 |

Table 15 lists the purity of each of the liquid preparations and lyophilized preparations after they have been placed for 14 days at 40°C. Each lyophilized preparation has a purity of 100% after being placed for 14 days at 40°C.

**Table 15**

| No. | Purity on the 0^{th} day % | Purity of liquid preparations after being placed for 14 days at 40°C % | Purity of lyophilized preparations after being placed for 14 days at 40°C % |
|---|---|---|---|
| Formulation 23 | 100 | 99.53 | 100 |
| Formulation 24 | 100 | 99.46 | 100 |
| Formulation 25 | 100 | 99.54 | 100 |
| Formulation 26 | 100 | 99.47 | 100 |
| Formulation 27 | 100 | 99.55 | 100 |
| Formulation 28 | 100 | 99.48 | 100 |

Table 16 lists the impurity content of each of the liquid preparations and lyophilized preparations after they had been placed for 14 days at 40°C.

**Table 16**

| No. | Placed for 14 days at 40°C | | | | | |
|---|---|---|---|---|---|---|
| | Liquid Preparations | | | Lyophilized Preparations | | |
| | Impurity A % | Impurity B % | Impurity C % | Impurity A % | Impurity B % | Impurity C % |
| Formulation 23 | Not detected | 0.469 | Not detected | Not detected | Not detected | Not detected |
| Formulation 24 | Not detected | 0.541 | Not detected | Not detected | Not detected | Not detected |
| Formulation 25 | Not detected | 0.457 | Not detected | Not detected | Not detected | Not detected |
| Formulation 26 | Not detected | 0.532 | Not detected | Not detected | Not detected | Not detected |
| Formulation 27 | Not detected | 0.448 | Not detected | Not detected | Not detected | Not detected |
| Formulation 28 | Not detected | 0.525 | Not detected | Not detected | Not detected | Not detected |

As shown in Table 16, Impurity B was generated in each of the liquid preparations. With the presence of 5% mannitol, 10% trehalose, or 10% sucrose, it is unexpected that each lyophilized preparation (100 mg/mL) of fosphenytoin sodium is free of Impurity A, Impurity B, Impurity C and other impurities after being placed for 14 days at 40°C, indicating that carbohydrates in lyophilized preparations can stabilize fosphenytoin sodium and avoid degradation.

### Experiment 4 Researches on lyophilization process

Different lyophilization process researches were performed on a pH 8.8 solution comprising 100 mg/mL fosphenytoin sodium, 5% mannitol and 100 mM tromethamine.

The formulation of fosphenytoin sodium liquid preparation for lyophilization is shown in Table 17 below.

**Table 17**

| Formulation Composition | Solution pH |
|---|---|
| 100 mg/mL fosphenytoin sodium, 100 mM tromethamine, 5% mannitol, water for injection, 1N HCl or NaOH solution | 8.8 |

Preparation method:
fosphenytoin sodium was dissolved in water for injection at 25°C, and tromethamine and mannitol were added to the solution; after stirring and dissolving, the pH was adjusted to 8.8-with 1N HCl or NaOH; water for injection was added to a constant volume of 100 mL, and filtering was performed with 0.2 µm PES membrane, as a result, a fosphenytoin sodium liquid preparation shown in the formulation was prepared; the liquid preparation was filled into a 15 mL vial at a 7.5 mL specification; and
the liquid preparations prepared in the afore-mentioned method were respectively lyophilized according to the process parameters in Table 18.

**Table 18**

| Process | Steps | Shelf Temperature | Heating Rate | Holding Time | Pressure | Total Lyophilization Time |
|---|---|---|---|---|---|---|
| #1 | Loading | ambient temperature | N/A | N/A | N/A | 95 h |
| | Pre-freezing | -50°C | -1 °C/min | 2 h | N/A | |
| | Annealing | -20°C | 1 °C/min | 1 h | N/A | |
| | Pre-freezing | -50°C | -1 °C/min | 2 h | N/A | |
| | Sublimiation drying | -20°C | 1 °C/min | 78 h | 70 mTorr | |
| | Analytical drying | 30°C | 1 °C/min | 12 h | 70 mTorr | |
| #2 | Loading | ambient temperature | N/A | N/A | N/A | 141 h |
| | Pre-freezing | -50°C | -1 °C/min | 3 h | N/A | |
| | Sublimiation drying | -30°C | 1 °C/min | 125 h | 70 mTorr | |
| | Analytical drying | 20°C | 1 °C/min | 13 h | 70 mTorr | |
| #3 | Loading | ambient temperature | N/A | N/A | N/A | 141 h |
| | Pre-freezing | -50°C | -1 °C/min | 3 h | N/A | |
| | Sublimiation drying | -30°C | 1 °C/min | 125 h | 70 mTorr | |
| | Analytical drying | 30°C | 1 °C/min | 13 h | 70 mTorr | |
| #4 | Loading | 4°C | -1 °C/min | 1 h | N/A | 142 h |
| | Pre-freezing | -50°C | -5 °C/min | 3 h | N/A | |
| | Sublimiation drying | -30°C | 1 °C/min | 128 h | 70 mTorr | |
| | Analytical drying | 30°C | 1 °C/min | 10 h | 70 mTorr | |
| #5 | Loading | ambient temperature | N/A | N/A | N/A | 51 h |
| | Pre-freezing | -50°C | -1 °C/min | 3 h | N/A | |
| | Drying | 20°C | 0.05 °C/min | 47 h | 70 mTorr | |
| #6 | Loading | ambient temperature | N/A | N/A | N/A | 44 h |
| | Pre-freezing | -50°C | -1.5 °C/min | 3 h | N/A | |
| | Drying | 20°C | 1.5 °C/min | 41 h | 75 mTorr | |

| | | | | | | |
|---|---|---|---|---|---|---|
| Note: the ambient temperature is 20°C. | | | | | | |

Table 19 shows the moisture content, reconstitution time and stability of the lyophilized samples obtained by different lyophilization processes.

**Table 19**

| Process | Mositure content of lyophilized samples on the 0^{th} day (%) | Reconstitution time of lyophilized samples on the 0^{th} day (s) | Impurity content after being placed for 14 days at 2°C to 8°C (%) | Impurity content after being placed for 14 days at 25°C (%) | Impurity content after being placed for 14 days at 40°C (%) |
|---|---|---|---|---|---|
| #1 | 2.50 | Failed to be completely reconstituted in one hour | Not detected | Not detected | Not detected |
| #2 | 3.23 | 55 s | Not detected | Not detected | Not detected |
| #3 | 2.47 | 40 s | Not detected | Not detected | Not detected |
| #4 | 2.85 | 45 s | Not detected | Not detected | Not detected |
| #5 | 3.39 | 40 s | Not detected | Not detected | Not detected |
| #6 | 3.60 | 27 s | Not detected | Not detected | Not detected |

The results in Table 18 and Table 19 show that the lyophilized products obtained by all lyophilization processes are cakes with a moisture content of 2.5% to 3.6%. No impurity was generated in all lyophilized products stored for 14 days at 2°C to 8°C, at 25°C and at 40°C.

The reconstitution time for all of the lyophilized products obtained by lyophilization processes #2 to #6 is within 1 minute, while the reconstitution time of the lyophilized product obtained by the lyophilization process #1 is longer. The total lyophilization time for conventional lyophilization processes (lyophilization processes #1 to #4) varies from about 4 days to 6 days. Unexpectedly, the lyophilization process #5 and the lyophilization process #6 of the present invention are very fast, and the lyophilization time is shortened to about 2 days. Relative to the lyophilization time of conventional lyophilization processes (#2, #3, #4), which is at least 141 hours, the lyophilization time of lyophilization processes (#5, #6) of the present disclosure was reduced by at least 60%, wherein the lyophilization time of lyophilization process #5 was reduced by about 64% and that of lyophilization process #6 was reduced by about 69%. (Reduced time of lyophilization process #5={ [(lyophilization time of lyophilization process #4-lyophilization time of lyophilization process #5)/lyophilization time of lyophilization process #4]×100%; reduced time of lyophilization process #6=[(lyophilization time of lyophilization process #4-lyophilization time of lyophilization process #6)/lyophilization time of lyophilization process #4]×100%}

Table 20 lists the product appearance, reconstitution time and pH of the lyophilized samples obtained in lyophilization process #6 after being placed for 0 days and 14 days under different conditions.

**Table 20**

| Samples | Lyophilization process | Lyophilized Preparations | | Reconstituted lyophilized preparation | |
|---|---|---|---|---|---|
| | | Appearance | Reconstitution time (s) | Appearance | pH |
| Lyophilized preparation on the 0^{th} day | #6 | white, slightly cracked, no collapse | 27 | clear, colorless | 8.95 |
| Lyophilized preparation placed for 14 days at 2°C to 8°C | #6 | white, slightly cracked, no collapse | 18 | clear, colorless | 8.99 |
| Lyophilized preparation placed for 14 days at 25°C | #6 | white, slightly cracked, no collapse | 21 | clear, colorless | 9.00 |
| Lyophilized preparation placed for 14 days at 40°C | #6 | white, slightly cracked, no collapse | 22.8 | clear, colorless | 8.98 |

As indicated by the results in Table 20, with respect to the lyophilized product prepared according to lyophilization process #6, the sample taken on the 0^{th} day is the same as those after being placed for 14 days respectively at 2°C to 8°C, 25°C and 40°C in appearance, which is in a shape of cake with no collapse. The lyophilized product was reconstituted into a 75 mg/mL fosphenytoin solution with water for injection, and the reconstitution time for all products was less than one minute. After reconstitution of the lyophilized product, the samples were clear, colorless and particle-free, and the pHs thereof were target pH of 8.8-9.0.

Table 21 lists RP-HPLC analysis results of the lyophilized samples obtained in the lyophilization process #6 on the 0^{th} day and after being placed for 14 days under different conditions.

**Table 21**

| Samples | Concentration (mg/mL) | Content % | Purity % | Impurity A % | Impurity B % | Impurity C % |
|---|---|---|---|---|---|---|
| Liquid preparation on the 0^{th} day | 97.2 | 97.2 | 100 | Not detected | Not detected | Not detected |
| Lyophilized preparation on the 0^{th} day | 74.3 | 99.1 | 100 | Not detected | Not detected | Not detected |
| Lyophilized preparation placed for 14 days at 2°C to 8°C | 70.6 | 94.1 | 100 | Not detected | Not detected | Not detected |
| Lyophilized preparation placed for 14 days at 25°C | 79.7 | 106.2 | 100 | Not detected | Not detected | Not detected |
| Lyophilized preparation placed for 14 days at 40°C | 79.6 | 106.2 | 100 | Not detected | Not detected | Not detected |

The results in Table 21 show that the preparations before and after lyophilization are free of impurities on the 0^{th} day, and the concentrations and contents all met the requirements of the United States Pharmacopoeia (USP 42). All lyophilized preparations placed for 14 days at 2°C to 8°C, at 25°C and at 40°C have a purity of 100%, and are free of Impurity A, Impurity B, Impurity C and other impurities.

### Experiment 5 Scaled-up lyophilization research

The formulation of fosphenytoin sodium liquid preparation for lyophilization is shown in Table 22 below.

**Table 22**

| Formulation Composition | Solution pH |
|---|---|
| 100 mg/mL fosphenytoin sodium, 100 mM tromethamine, 5% mannitol, water for injection, a pH regulator | 8.8 |

### Solution preparation before lyophilization:

Fosphenytoin sodium was dissolved in water for injection at 40°C, and tromethamine and mannitol were added to the solution; after stirring and dissolving, the pH was adjusted to 8.8 with 1N HCl or NaOH; water for injection was added to a constant volume of 1 L, and filtering was performed with 0.2 µm PES membrane, as a result, a fosphenytoin sodium liquid preparation with a concentration shown in the formulation was prepared.

### Scaled-up lyophilization process #7-1

Solution before lyophilization was filled into a 5 mL vial at a 1.5 mL specification; and lyophilization was performed according to the process parameters shown in Table 23.

**Table 23**

| Steps | Shelf Temperature | Heating Rate (°C/min) | Holding Time (h) | Pressure |
|---|---|---|---|---|
| Loading | ambient temperature | N/A | N/A | N/A |
| Pre-freezing | -50°C | -1.5 °C/min | 3 | N/A |
| Drying | 20°C | 1.5 °C/min | 27 | 75 mTorr |
| secondary drying | 30°C | 1.5 °C/min | 12 | 75 mTorr |

| | | | | |
|---|---|---|---|---|
| Ambient temperature: 20°C | | | | |

### Scaled-up lyophilization process #7-2

Solution before lyophilization was filled into a 15 mL vial at a 7.5 mL specification; and lyophilization was performed according to the process parameters shown in Table 24.

**Table 24**

| Steps | Shelf Temperature | Heating Rate (°C/min) | Holding Time (h) | Pressure |
|---|---|---|---|---|
| Loading | ambient temperature | N/A | N/A | N/A |
| Pre-freezing | -50°C | -1.5 °C/min. | 3 | N/A |
| Drying | 20°C | 1.5 °C/min | 35 | 75 mTorr |
| secondary drying | 30°C | 1.5 °C/min | 12 | 75 mTorr |

| | | | | |
|---|---|---|---|---|
| Ambient temperature: 20°C | | | | |

The lyophilization process was operated in a way as follows.

Samples were loaded at 20°C, and the shelf temperature was lowered to -50°C at a rate of 1.5 °C/min for pre-freezing. The preparations were frozen for 3 hours at -50°C. The lyophilizer pressure was set to 75 mTorr, and then the shelf temperature was raised to 20°C at a heating rate of 1.5 °C/min. Once the Pirani pressure reached the set pressure, the shelf temperature was raised to 30°C at a heating rate of 1.5 °C/min. The data indicated that the lyophilization process was completed in less than 2 days for samples prepared according to scaled-up lyophilization process #7-1 (with a loading volume of 1.5 mL). The lyophilization process was completed in less than 3 days for samples prepared according to scaled-up lyophilization process #7-2 (with a loading volume of 7.5 mL).

### Temperature and pressure curves for scaled-up lyophilization processes

The temperature and pressure curves of scaled-up lyophilization processes #7-1 and 7-2 are shown in Figs. 6-9.

For samples with a loading volume of 1.5 mL, the Pirani pressure reached 75 m torr after 20 hours, and a secondary drying was performed at 30°C after drying of 30 hours. More moisture was released during the secondary drying at 30°C, as evidenced by the increase in Pirani pressure.

For samples with a loading volume of 7.5 mL, the Pirani pressure reached 75 m torr after 30 hours, and a secondary drying was performed at 30°C after drying of 38 hours. More moisture was released during the secondary drying at 30°C, as evidenced by the increase in Pirani pressure.

The temperature curve of the scaled-up lyophilization processes 7-1 and 7-2 show that, during the drying process at -20°C, the product with a loading volume of 1.5 mL was kept for about 4 hours and the product with a loading volume of 7.5 mL was kept for about 10 hours.

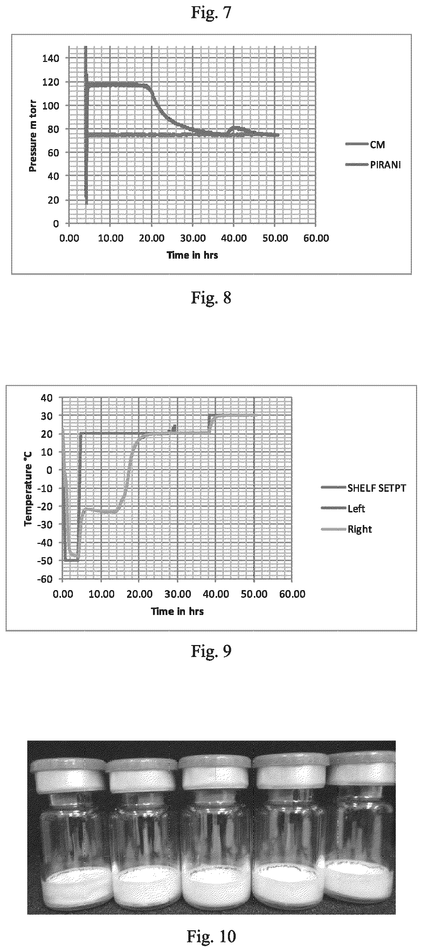

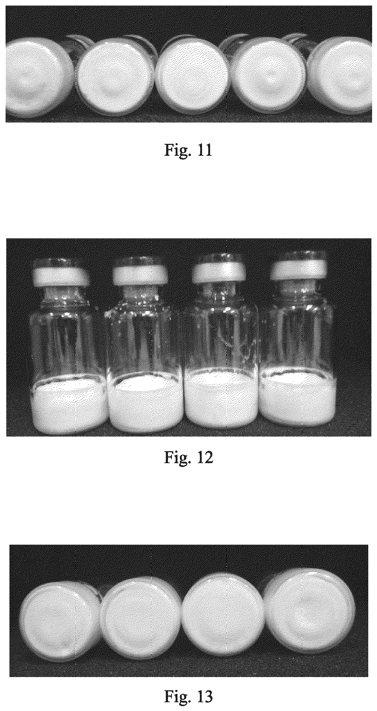

In the scaled-up lyophilization process, the lyophilized sample on the 0^{th} day was a white, slightly cracked cake with no collapse (Figs. 10 to 13), the reconstitution time for the sample was about 56 to 70 seconds, and the moisture content was about 2% to 3%. After the lyophilized samples had been placed for 1 month at 2°C to 8°C, at 25°C and at 40°C, they were still white and slightly cracked cakes, wherein: samples with a loading volume of 7.5 mL had a moisture content of about 3% while samples with a loading volume of 1.5 mL had a moisture content of about 3% to 6%, and the reconstitution time of the lyophilized samples was about 31 to 88 seconds (Table 25).

**Table 25**

| Samples | Lyophilization | Filling Volume | Appearance of | Reconstitution | Moisture |
|---|---|---|---|---|---|
| | Process | mL | Lyophilized Preparation | Time (s) | content % |
| Lyophilized sample on the 0^{th} day | #7-1 | 1.5 | white, slightly cracked, no collapse | 70 | 2.17 |
| | #7-2 | 7.5 | white, slightly cracked, no collapse | 56 | 3.36 |
| Lyophilized sample after being placed for 1 month at 2°C to 8°C | #7-1 | 1.5 | white, slightly cracked, no collapse | 56 | 3.88 |
| | #7-2 | 7.5 | white, slightly cracked, no collapse | 31 | 3.27 |
| Lyophilized sample after being placed for 1 month at 25°C | #7-1 | 1.5 | white, slightly cracked, no collapse | 81 | 6.67 |
| | #7 -2 | 7.5 | white, slightly cracked, no collapse | 33 | 3.01 |
| Lyophilized sample after being placed for 1 month at 40°C | #7-1 | 1.5 | white, slightly cracked, no collapse | 88 | 2.60 |
| | #7-2 | 7.5 | white, slightly cracked, no collapse | 41 | 3.00 |

The lyophilized preparation was reconstituted in water for injection to obtain a fosphenytoin sodium solution with a concentration of 75 mg/mL, wherein pH of the reconstituted sample taken on the 0^{th} day is 8.8 to 8.9, which is not significantly different from that of the dissolved samples placed for 1 month at 2°C to 8°C, at 25°C and at 40°C (Table 26).

**Table 26**

| Samples | Lyophilization Process | Filling Volume (mL) | Appearance of Reconstituted Preparation | pH of Reconstituted Preparation |
|---|---|---|---|---|
| Lyophilized sample on the 0^{th} day | #7-1 | 1.5 | clear, colorless | 8.91 |
| | #7-2 | 7.5 | clear, colorless | 8.94 |
| Lyophilized sample after being placed for 1 month at 2°C to 8°C | #7-1 | 1.5 | clear, colorless | 8.90 |
| | #7-2 | 7.5 | clear, colorless | 8.85 |
| Lyophilized sample after being placed for 1 month at 25°C | #7-1 | 1.5 | clear, colorless | 8.88 |
| | #7-2 | 7.5 | clear, colorless | 8.84 |
| Lyophilized sample after being placed for 1 month at 40°C | #7-1 | 1.5 | clear, colorless | 8.87 |
| | #7-2 | 7.5 | clear, colorless | 8.83 |

Table 27 shows the RP-HPLC analysis results of the preparations obtained from scaled-up lyophilization processes #7-1 and #7-2. Liquid and lyophilized preparations on the 0^{th} day were free of Impurity A and Impurity B. The preparations were at target concentrations.

**Table 27**

| Lyophilized Preparations on the 0^{th} Day | | | |
|---|---|---|---|
| Samples | Concentration (mg/mL) | Content% | Purity% |
| Liquid Preparations | 100.2 | 100.2 | 100.0 |
| 1.5 mL lyophilized preparation (Process #7-1) | 80.7 | 107.6 | 100.0 |
| 7.5 mL lyophilized preparation (Process #7-2) | 80.7 | 107.7 | 100.0 |

Tables 28-30 show the RP-HPLC analysis results of lyophilized preparations placed for 1 month, 2 months and 3 months at 2°C to 8°C, at 25°C and at 40°C. The data indicated that no impurities were generated after the lyophilized preparation had been placed for 3 months at 2°C to 8°C, at 25°C and at 40°C.

**Table 28**

| Scaled-up lyophilization process: lyophilized vial placed for 1 month under different conditions (1.5 mL lyophilized preparation, scaled-up process #7-1; 7.5 mL lyophilized preparation, scaled-up process #7-2) | | | | | |
|---|---|---|---|---|---|
| Samples | Content % | Purity % | Impurity A % | Impurity B % | Impurity C % |
| 1.5 mL lyophilized preparation, 2°C to 8°C | 97.1 | 100.0 | Not detected | Not detected | Not detected |
| 7.5 mL lyophilized preparation, 2°C to 8°C | 100.4 | 100.0 | Not detected | Not detected | Not detected |
| 1.5 mL lyophilized preparation, 25°C | 104.5 | 100.0 | Not detected | Not detected | Not detected |
| 7.5 mL lyophilized preparation, 25°C | 101.5 | 100.0 | Not detected | Not detected | Not detected |
| 1.5 mL lyophilized preparation, 40°C | 109.4 | 100.0 | Not detected | Not detected | Not detected |
| 7.5 mL lyophilized preparation, 40°C | 102.2 | 100.0 | Not detected | Not detected | Not detected |

**Table 29**

| | | | | | |
|---|---|---|---|---|---|
| Scaled-up lyophilization process: lyophilized vial placed for 2 months under different conditions (1.5 | mL | | | | |

| lyophilized preparation, scaled-up process #7-1; 7.5 mL lyophilized preparation, scaled-up process #7-2) | | | | | |
|---|---|---|---|---|---|
| Samples | Content % | Purity % | Impurity A % | Impurity B % | Impurity C % |
| 1.5 mL lyophilized preparation, 2°C to 8°C | 104.2 | 100.0 | Not detected | Not detected | Not detected |
| 7.5 mL lyophilized preparation, 2°C to 8°C | 100.7 | 100.0 | Not detected | Not detected | Not detected |
| 1.5 mL lyophilized preparation, 25°C | 105.9 | 100.0 | Not detected | Not detected | Not detected |
| 7.5 mL lyophilized preparation, 25°C | 96.3 | 100.0 | Not detected | Not detected | Not detected |
| 1.5 mL lyophilized preparation, 40°C | 101.4 | 100.0 | Not detected | Not detected | Not detected |
| 7.5 mL lyophilized preparation, 40°C | 95.7 | 100.0 | Not detected | Not detected | Not detected |

**Table 30**

| Scaled-up lyophilization process: lyophilized vial placed for 3 months under different conditions (1.5 mL lyophilized preparation, scaled-up process #7-1; 7.5 mL lyophilized preparation, scaled-up process #7-2) | | | | | |
|---|---|---|---|---|---|
| Samples | Content % | Purity % | Impurity A % | Impurity B % | Impurity C % |
| 1.5 mL lyophilized preparation, 2°C to 8°C | 102.9 | 100.0 | Not detected | Not detected | Not detected |
| 7.5 mL lyophilized preparation, 2°C to 8°C | 99.5 | 100.0 | Not detected | Not detected | Not detected |
| 1.5 mL lyophilized preparation, 25°C | 99.5 | 100.0 | Not detected | Not detected | Not detected |
| 7.5 mL lyophilized preparation, 25°C | 101.9 | 100.0 | Not detected | Not detected | Not detected |
| 1.5 mL lyophilized preparation, 40°C | 104.0 | 100.0 | Not detected | Not detected | Not detected |
| 7.5 mL lyophilized preparation, 40°C | 101.8 | 100.0 | Not detected | Not detected | Not detected |

## Claims

1. A fosphenytoin sodium solid composition, comprising fosphenytoin sodium and at least one carbohydrate.

2. The fosphenytoin sodium solid composition according to claim 1, wherein the solid composition can be stored at room temperature.

3. The fosphenytoin sodium solid composition according to claim 1 or 2, wherein the solid composition is a lyophilized composition.

4. The fosphenytoin sodium solid composition according to any one of claims 1 to 3, wherein the carbohydrate is at least one selected from sugar and oligosaccharide;
preferably, the sugar is at least one selected from monosaccharide, disaccharide and sugar alcohol;
preferably, the monosaccharide is at least one selected from glucose, galactose and fructose;
preferably, the disaccharide is at least one selected from sucrose, lactose, trehalose, maltose and isomaltose;
preferably, the sugar alcohol is at least one selected from sorbitol, mannitol, xylitol and maltitol; and
preferably, the oligosaccharide is at least one selected from raffinose, stachyose, isomaltooligosaccharide, fructooligosaccharide, mannose oligosaccharide and soybean oligosaccharide.

5. The fosphenytoin sodium solid composition according to claim 3 or 4, wherein, prior to lyophilization, the weight-to-volume ratio of the carbohydrate in the composition is 1% to 20%, preferably 3% to 15%, and more preferably 5% to 10%.

6. The fosphenytoin sodium solid composition according to any one of claims 1 to 4, wherein the solid composition further comprises a buffer.

7. The fosphenytoin sodium solid composition according to claim 6, wherein the buffer is one or more selected from phosphate buffer, hydrophosphate buffer, dihydrogen phosphate buffer, bicarbonate buffer, carbonate buffer, boric acid buffer, borate buffer, amino acid buffer, trialkylamine buffer, tromethamine buffer, pyrophosphate buffer, Glycyl Glycine (glycylglycine) buffer; preferably, the phosphate, hydrophosphate, dihydrogen phosphate, bicarbonate, carbonate, borate and pyrophosphate are independently a sodium salt and/or a potassium salt; the trialkylamine is trimethylamine; preferably, prior to lyophilization, a concentration of the buffer is 10 to 150 mM, and more preferably 20 to 100 mM.

8. The fosphenytoin sodium solid composition according to any one of claims 1 to 7, wherein the pH of the fosphenytoin sodium solid composition prior to lyophilization or after reconstitution is 8 to 10, preferably 8 to 9.3, and more preferably 8 to 9.

9. The fosphenytoin sodium solid composition according to any one of claims 1 to 8, wherein, prior to lyophilization, the concentration of the fosphenytoin sodium is 75 mg/mL to 150 mg/mL, preferably 75 mg/mL to 100 mg/mL; and more preferably 75 mg/mL or 100 mg/mL.

10. The fosphenytoin sodium solid composition according to any one of claims 1 to 9, wherein, after reconstitution, the concentration of the fosphenytoin sodium is 75 mg/mL to 150 mg/mL, preferably 75 mg/mL to 100 mg/mL; and more preferably 75 mg/mL.

11. The fosphenytoin sodium solid composition according to any one of claims 1 to 10, wherein no Impurity A, Impurity B or Impurity C is generated in the fosphenytoin sodium solid composition after being placed at 25°C and 60% relative humidity for 14 days; preferably, no Impurity A, Impurity B or Impurity C is generated in the fosphenytoin sodium solid composition after being placed at 25°C and 60% relative humidity for 3 months,

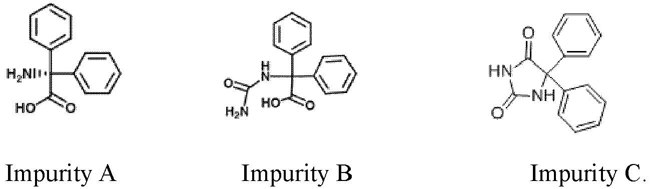

12. The fosphenytoin sodium solid composition according to any one of claims 1 to 11, wherein no Impurity A, Impurity B or Impurity C is generated in the fosphenytoin sodium solid composition after being placed at 40°C and 75% relative humidity for 14 days; preferably, no Impurity A, Impurity B or Impurity C is generated in the fosphenytoin sodium solid composition after being placed at 40°C and 75% relative humidity for 3 months,

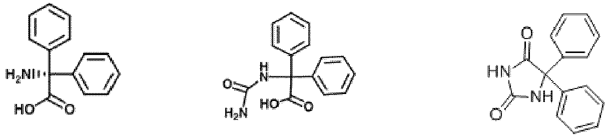

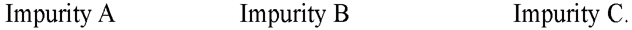

13. The fosphenytoin sodium solid composition according to any one of claims 1 to 12, wherein the solid composition is a lyophilized composition comprising fosphenytoin sodium, a buffer and at least one carbohydrate; the buffer is tromethamine; the carbohydrate is selected from trehalose, sucrose, mannitol or lactose; and the pH of the lyophilized composition before lyophilization or after reconstitution is 8 to 9.

14. The fosphenytoin sodium solid composition according to claim 13, wherein, prior to lyophilization, the concentration of the fosphenytoin sodium is 75 mg/mL or 100 mg/mL, the concentration of the buffer is 20 to 100 mM, and the weight-to-volume ratio of the carbohydrate in the composition is 5% to 10 %.

15. A lyophilization method of fosphenytoin sodium, wherein the total lyophilization time of the lyophilization method is reduced by more than 60% relative to a total lyophilization time of conventional lyophilization methods, preferably reduced by more than 70%.

16. The lyophilization method of fosphenytoin sodium according to claim 15, including: (1) preparing a fosphenytoin sodium solution; (2) pre-freezing the fosphenytoin sodium solution; and (3) directly increasing the shelf temperature at a certain heating rate to a predetermined temperature for drying, rather than individually setting a sublimation drying step and an analytical drying step.

17. The lyophilization method of fosphenytoin sodium according to claim 16, wherein: the fosphenytoin sodium solution comprises fosphenytoin sodium and at least one carbohydrate; preferably, the carbohydrate is at least one selected from sugar and oligosaccharide, and the sugar is selected at least one from monosaccharide, disaccharide and sugar alcohol; preferably, the monosaccharide is at least one selected from glucose, galactose and fructose; preferably, the disaccharide is at least one selected from sucrose, lactose, trehalose, maltose and isomaltose; preferably, the sugar alcohol is at least one selected from sorbitol, mannitol, xylitol and maltitol; preferably, the oligosaccharide is at least one selected from affinose, stachyose, isomaltooligosaccharide, fructooligosaccharide, mannose oligosaccharide and soybean oligosaccharide; preferably, the weight-to-volume ratio of the carbohydrates is 1% to 20%, preferably 3% to 15%, and more preferably 5% to 10%; preferably, the pH value of the fosphenytoin sodium solution is 8 to 10, preferably 8 to 9.3, and more preferably 8 to 9; preferably, the fosphenytoin sodium solution further comprises a buffer, and, preferably, the buffer is one or more selected from phosphate buffer, hydrophosphate buffer, dihydrogen phosphate buffer, bicarbonate buffer, carbonate buffer, boric acid buffer, borate buffer, amino acid buffer, trialkylamine buffer, tromethamine buffer, pyrophosphate buffer, Glycyl Glycine (glycylglycine) buffer; preferably, the phosphate, hydrophosphate, dihydrogen phosphate, bicarbonate, carbonate, borate, pyrophosphate are independently a sodium salt and/or a potassium salt; the trialkylamine is trimethylamine; preferably, the concentration of the buffer is 10 to 150 mM, and more preferably 20 to 100 mM; preferably, the concentration of the fosphenytoin sodium is 75 mg/mL to 150 mg/mL; more preferably 75 mg/mL to 100 mg/mL; and further preferably 75 mg/mL or 100 mg/mL.

18. The lyophilization method of fosphenytoin sodium according to claim 16 or 17, wherein the pre-freezing temperature in Step (2) is -40°C to -60°C, preferably -45°C to -55°C, and more preferably -45°C to -50°C; preferably, the cooling rate of decreasing the temperature to the pre-freezing temperature is 0.5 to 6 °C/min, preferably 1 to 5 °C/min, and more preferably 1 to 1.5 °C/min; preferably, the heating rate in Step (3) is 0.01 to 5 °C/min, preferably 0.025 to 3 °C/min, and more preferably 0.05 to 1.5 °C/min; preferably, the shelf temperature is directly increased to 5°C to 25°C, preferably to 10°C to 25°C, and more preferably to 20°C to 25°C.

19. Use of the fosphenytoin sodium solid composition according to any one of claims 1 to 14 in preparation of a medicament for treatment of epilepsy or other convulsive states.
